# EUROPEAN PATENT SPECIFICATION

(11) **EP 2 588 066 B1**
(45) Date of publication and mention of the grant of the patent: **21.03.2018**
(21) Application number: 11738313.3
(22) Date of filing: 27.06.2011
(51) Int. Cl.: A61K 8/11, A61K 8/65, A61Q 19/00, A61K 8/92, A23L 33/10, A23L 33/115, A61Q 13/00

(54) **SOLID CORE COACERVATED CAPSULES**
KOAZERVIERTE KAPSELN MIT FESTEM KERN
CAPSULES COACERVÉES À NOYAU SOLIDE

(30) Priority: 30.06.2010 EP 10167816
(43) Date of publication of application: 08.05.2013
(73) Proprietor: Firmenich SA, 1211 Geneva 8 (CH)
(72) Inventor: DARDELLE, Gregory, CH-1211 Geneva 8 (CH)
(74) Representative: Rupp, Christian
(86) International application number: PCT/IB2011/052807
(87) International publication number: WO 2012/001604

(56) References cited:
- EP-A1- 0 316 054
- EP-A1- 0 455 598
- WO-A1-89/04714
- WO-A1-2008/134908
- WO-A2-2008/007234
- SHINZO OMI ET AL: "MICROENCAPSULATION OF PHEROMONE-ANALOGUE AND MEASUREMENT OF THE SUSTAINED RELEASE", JOURNAL OF MICROENCAPSULATION, TAYLOR AND FRANCIS, BASINGSTOKE, GB, vol. 8, no. 4, 1 October 1991 (1991-10-01) , pages 465-478, XP000232590, ISSN: 0265-2048

## Description

### Technical Field

The present invention relates to coacervated capsules for use as a delivery system and particularly relates to coacervated capsules comprising a solid core for use in the flavor and fragrance industry.

### Background Art

Coacervation, also called aqueous phase separation, is a very well known technique for encapsulating hydrophobic liquids. The process provides oil-containing microcapsules, the encapsulating material being a gelled hydrophilic colloid that is impervious to the oil and deposited evenly and densely around the oil. The encapsulating material is a protein which may be complexed with another colloid having an opposite electric charge.

A coacervation process may be "simple" or "complex". The former designation is employed when a single protein is used to form a capsule wall as phase separation is taking place. The latter term designates the use of a second oppositely charged non-protein polymer to bring about phase separation. Complex coacervation method is widely practiced in commercial processes and has been well described in the literature. In particular US 2,800,457 and US 2,800,458 disclose complex coacervation in a very detailed manner.

Generally, a coacervation process comprises four basic steps consisting of, respectively, emulsification, phase separation, wall formation and wall hardening. In a complex coacervation process the wall surrounding the core material is, as mentioned above, constituted of two oppositely charged high molecular weight colloids. In most of the cases, the positively charged colloid used is a gelatin, a functional protein derived from collagen by hydrolysis and subsequent extraction.

In the case of coacervation, the core of the core-shell is typically a liquid at room temperature since the processing steps are more easily achieved with liquid active ingredients, such as liquid perfumes and liquid flavors. Nevertheless, upon storage of such encapsulation systems often suffers the drawback of leakage of the core from the shell. It would be advantageous to address this problem.

WO-A1-2008/134908 (Givaudan) discloses microcapsules having a shell and a core wherein the core comprises a waxy solid. The preferred waxy solid, beeswax, forms a dispersion of particles in a water insoluble liquid which contains or is itself an active material. This system is described as having a crystalline structure and a consistency that is semi-solid. Whilst such a system slows the movement of liquid through its high viscosity (semi-solid consistency), this does not ensure little or no leakage of the liquid. It would be desirable to address this.

Accordingly, it would be advantageous to improve the stability, for instance to offer a better stability in a surfactant solution by limiting the diffusion of small molecules from the core. It would also be desirable to improve the mechanical properties, for instance to provide capsules that are more resistant than typical coacervated capsules to handling and transport at room temperature of standard core-shell capsules made by complex coacervation.

WO-A-2009/046930 (Cognis) describes the formation of wax microcapsules in which an active ingredient is mixed into the melted wax and the wax cooled thereby entrapping the active ingredient. There is no mention or suggestion of complex coacervation systems and so this document does not contemplate the use of such a wax-encapsulated system in an environment where a coacervate system would remain stable but the wax system would not, such as at higher temperatures or in harsh environments, e.g. containing large amounts of surfactant.

EP-A1-0316054 (Shiseido Co Ltd) discloses microcapsules comprising a gelatin film swollen with water. A polyhydric alcohol is used in a cosmetic base to modify the strength of the capsule membrane. In the examples, the initial breaking strength is systematically decreased when the capsule is dispersed in polyhydric alcohol mixtures. By contrast, it would be desirable to provide capsules that maintain a high breaking strength.

In the article by Shinzo Omi et al, "Microencapsulation of Pheromone-Analogue and Measurement of the Sustained Release", Journal of Microencapsulation, Taylor and Francis, vol 8, no. 4, 1991 pages 465 to 476, a coacervate system is described containing a core of 90% wax and 10% of the active ingredient. This provides a product having a high melting point in the order of 65°C or higher. It would be desirable to have a very high loading of the active ingredient.

In WO-A2-2008/007234 (Firmenich) fat is encapsulated in a coacervate system. In a foodstuff, the encapsulated fat improves the mouthfeel when consumed. The addition of flavor compounds is limited to very low levels since the document is concerned with mouthfeel enhancement. The fat exemplified, namely tallow, has a melting point of about 15°C, which is far below a melting point that maintains a solid core at room temperature and which liquefies at or about 37°C (the typical temperature in the oral cavity.

The present invention thus seeks to address one or more of the abovementioned problems.

### Summary of the Invention

Thus, the present invention provides A coacervated capsule comprising:
(a) from 10 to 99% by weight of the capsule of a core comprising a mixture of (I) a fatty component comprising (i) hydrogenated oil or (ii) hydrogenated fat or (iii) cocoa butter or (iv) a mixture thereof, and (II) a material to be encapsulated comprising a flavor and/or fragrance material, the mixture having a Tm of between about 30°C and about 40°C such that it is a solid at 20°C, wherein the weight ratio of fatty component to material to be encapsulated is 50:50 and
(b) from 90 to 1% by weight of the capsule of a coating layer comprising essentially a protein, and optionally a non-protein polymer.

The invention further provides a process for the preparation of stable coacervated capsules comprising the step of:
(a) preparing a core mixture comprising:
   (I) a fatty component comprising (i) hydrogenated oil or (ii) hydrogenated fat or (iii) cocoa butter or (iv) a mixture thereof and, and
   (II) a material to be encapsulated comprising a flavor and/or fragrance material, the core having a Tm of about 30°C and about 40°C such that it is a solid at room temperature;
(b) providing an aqueous solution comprising a protein and, optionally a non-protein;
(c) mixing the core mixture and the aqueous solution to form an emulsion or suspension;
(d) inducing phase separation such that the protein and, optionally a non-protein polymer, form a wall around the core mixture; and
(e) optionally cross-linking the wall.

### Brief Description of the Drawings

**Figures 1a and 1b** show coacervated capsules according to the invention in an aqueous solution of sodium dodecyl sulphate at time, t=0, and time, t=2 hours, respectively;
**Figures 2a and 2b** show comparative coacervated capsules where the core component has a melting point below room temperature at time, t=0, and time, t=2 hours, respectively; and
**Figures 3a and 3b** show comparative coacervated capsules in an aqueous solution of SDS at time, t=0 and time, t=2 hours respectively.

### Detailed Description

### Core Mixture

The core of the coacervated capsule comprises an active ingredient. The active ingredient is a flavor and/or a fragrance.

At 20°C, the active ingredient may be in the liquid or solid state, though it is usually a liquid. Preferably the active ingredient is a hydrophobic material, by which it is meant that the active ingredient is not miscible in demineralised water at 25°C and, when added to it, form a separate, hydrophobic phase.

The terms "flavors" and "fragrances" as used herein are deemed to define a variety of flavor and fragrance materials of both natural and synthetic origins. They include single compounds or mixtures. Specific examples of such components may be found in the literature, e.g. in Fenaroli's Handbook of Flavor Ingredients, 1975, CRC Press; synthetic Food Adjuncts, 1947 by M.B. Jacobs, edited by van Nostrand; or Perfume and Flavor Chemicals by S. Arctander 1969, Montclair, N.J. (USA). These substances are well known to the person skilled in the art of perfuming, flavoring and/or aromatising consumer products, i.e. of imparting an odour and/or flavor to a consumer product traditionally perfumed or flavored, or of modifying the odour of the consumer product.

The flavor may be a taste modifier. "Taste modifier" denotes an active ingredient that operates on a consumer's taste receptors or provides a mouthfeel such as body or roundness to a product being consumed. Non-limiting examples of taste modifiers include active ingredients that enhance, modify or impart sweetness, acidity, tingling, bitterness, sourness, saltiness, fattiness, umami, kokumi, heat sensation or cooling sensation.

The fragrance and/or flavor preferably comprises at least 5 wt.%, more preferably at least 10 wt.%, even more preferably at least 20wt.%, most preferably at least 30wt.%, e.g. at least 40 wt.% of chemical compounds having a vapour pressure of ≥ 0.007 Pa at 25°C. Preferably, at least 10wt.% have a vapour pressure of ≥ 0.1, more preferably, at least 10wt.% have a vapour pressure of ≥ 1 Pa at 25°C, and most preferably, at least 10wt.% have a vapour pressure of ≥ 10 Pa at 25°C. The value of 0.007 Pa at 25°C is selected because compounds meeting these criteria are generally regarded as having a volatile character. The present invention thus allows for efficient encapsulation of more volatile ingredients being present in high amounts relative to the total amount of active ingredient.

For the purpose of the present invention, the vapour pressure is determined by calculation using the method disclosed in "EPI suite"; 2000 U.S. Environmental Protection Agency.

The fragrance compound limonene is adduced for illustrating the determination of vapour pressure by calculation. By applying the method "EPI suite", limonene is calculated to have a vapour pressure of about 193 Pa at 25°C.

A fatty component is present as part of the core.

The fatty component comprises (i) a hydrogenated oil or (ii) a hydrogenated fat or (iii) cocoa butter or (iv) a mixture thereof. Suitable hydrogenated oils include hydrogenated palm oil, hydrogenated soybean oil and hydrogenated cottonseed oil. A suitable hydrogenated fat is, for example cocoa fat.

The presence of the fatty component provides the internal phase of the mixture undergoing coacervation to form a solid at the desired temperature, i.e. between about 30°C and about 40°C, and provides a solid core at room temperature. By room temperature, it is meant herein a temperature of 25°C.

Preferably the weight ratio of fatty component to the active ingredient is from 10:90 to 70:30. More preferably the weight ratio is in the range of from 30:70 to 50:50.

Without wishing to be bound by theory, the internal phase (that forms the core of the coacervate core-shell) has to be encapsulated in its liquid form. During the coacervation process, membrane formation typically starts at a temperature below 37°C. Thus, the Tm of the mixture of hydrogenated fat component and active ingredient has to be below 40°C to ensure that the hydrophobic phase remains liquid when membrane deposition starts.

If the Tm of the internal phase is higher than 40°C, membrane deposition will occur on a solid phase. By contrast, during storage, if the temperature exceed the Tm of the internal phase, then the volume increase between the solid and the liquid state will cause the capsule wall to break and release the active ingredient.

In the event that the active ingredient is a solid then, together with the hydrogenated fatty component, there is formed a suspension of the solid particles in the fatty component. In such a case, the fatty component itself must have a Tm of between about 30°C and about 40°C to provide the necessary physical characteristics to the core. In such a case, it is preferred that the viscosity, measured at ambient temperature, of the core droplet containing the suspension is from about 10 mPa.s to 1'000 mPa.s. More preferably the viscosity is from 100 mPa.s to 800 mPa.s, even more preferably from 200 mPa.s to 750 mPa.s. Above 1'000 mPa.s, it is found that the mixture is too viscous for the requirements of a typical coacervation process.

On the other hand, where the active material is a liquid, especially a flavor and/or perfume, it is found that the presence of the flavor and/or fragrance serves to decrease the Tm of the mixture containing the hydrogenated fatty component. The fat or the mixture of fats is thus simply selected by the skilled person so that the final mixture of the fatty component and the flavor and/or fragrance has a Tm between about 30°C and about 40°C.

A preferred fatty component is a mixture of a fat and a hydrogenated oil. Particularly preferred is a mixture of hydrogenated oil with coco fat and/or cocoa butter.

For instance, it is found that the combination of hydrogenated coco fat and hydrogenated palm oil, especially in a weight ratio of 1:3 to 1:1, typically provides a Tm of between about 30°C and about 40°C when mixed at a weight ratio of about 1:1 with a volatile flavor or perfume composition.

Where the active ingredient is a liquid, the droplets of the internal phase form an emulsion during the coacervation process. To form the emulsion, any suitable process known to the skilled person for performing the emulsification can be used. For example, preparation using a 4-bladed impeller shearing device operated at 300s⁻¹, the ratio of the diameter of the container to the diameter of the blades being about 2:1, would be suitable for the purposes of the present invention.

### Coating Layer

The coating layer, also known as the shell of the core-shell coacervate comprises a protein and, optionally, a non-protein polymer and forms a coacervate around the hydrophobic droplet. Preferably, the non-protein polymer is charged oppositely to the protein.

These materials are also referred to commonly as hydrocolloids, that is polymeric substances that can be dissolved in water, optionally at elevated temperatures, e.g. up to 90°C. These encompass polymers such as proteins, polysaccharides and polyacids, for example, that are generally known to be useful in coacervation methods.

The present invention encompasses "simple" and "complex" coacervation. In simple coacervation, protein alone is used to form a capsule wall as phase separation is taking place. Complex coacervation refers to methods in which a generally oppositely charged non-protein polymer and a protein polymer together form the capsule wall. According to the principles of complex coacervation the present invention method provides the optional addition of an oppositely charged non-protein polymer, preferably a polysaccharide, to the hydrocolloid solution.

Proteins useful in coacervation processes include albumins, vegetable globulins and gelatines. The molecular weight of the protein is typically in the order of 40'000 to 500'000 preferably 20'000 to 250'000. Some protein aggregates, however, may have molecular weights even greater than this.

Preferably, the protein is a gelatine. It is preferable to use gelatine having good physicochemical and chemical properties as typified by good film forming ability, amphoteric properties, the controllability of the quantity of charges by pH, and, preferably, the occurrence of the change from solution to gel at a critical temperature. Stated specifically, any gelatine that satisfies the specification for use in production of microcapsules may be employed.

The gelatine may be fish, pork, beef, and/or poultry gelatine, for example. According to a preferred embodiment, the protein is fish, beef or poultry gelatine. According to a more preferred embodiment, the protein is warm water fish gelatine. Preferably, the warm water fish gelatine has a bloom of from about 150 to about 300 bloom, more preferably from about 200 to about 300 bloom. Preferably, the warm water fish gelatine has ≥ 250 bloom. According to the general knowledge, warm water fish are fish that are capable of tolerating water above 27°C over prolonged time.

Typical non-protein polymers useful in complex coacervation methods include, in particular, negatively charged polymers. For example, they may be selected from gum arabic, xanthan, agar, alginate salts, cellulose derivatives, for example carboxymethyl cellulose, pectinate salts, carrageenan, polyacrylic and methacrylic acid, and/or mixtures thereof. Further suitable non-proteins can be derived from the literature, for example from WO 2004/022221, page 4, lines 27-29.

The protein and, optionally, non-protein polymers are usually dissolved in water to form a hydrocolloid solution. Preferably, in the aqueous hydrocolloid solution, the protein is present in an amount of from 0.5 to 3.5wt%, more preferably from 1 to 2wt%.

If present, the amount of polysaccharide is preferably from 0.5 to 3.5wt%, more preferably from 1 to 2% wt.% in the aqueous solution.

In a particular embodiment, the weight ratio between the protein and the non-protein polymer is from about 3:1 to 1:3, more preferably 2:1 to 1:1, most preferably about 3:2.

### Cross-linking agent

A cross-linking agent is typically used to harden the coating layer. Suitable cross-linking agents include formaldehyde, acetaldehyde, glutaraldehyde, glyoxal, chrome alum, or transglutaminase. Preferably, transglutaminase is used at 10-100, preferably 30-60 activity units per gram of gelatine. This enzyme is well described and commercially obtainable.

### Preparation

According to a preferred embodiment, the coacervated capsule is prepared by forming a first solution of the protein material above its gelling temperature and a second aqueous solution of the non-protein polymer. The two solutions are mixed to form a third solution.

The active ingredient is then mixed with the fatty component (this mixture will eventually form the internal phase) and introduced into the third solution under shear to form an emulsion or suspension. The emulsion and/or suspension may be prepared in a conventional manner. Preferably, the internal phase mixture is slowly added to the third solution over a period of about 3 to 10 minutes, preferably 4 to 6 minutes, with a stirrer being adjusted to 300 to 400 rpm. The stirrer speed can be adjusted as desired.

In an alternative embodiment, the emulsion can be prepared by membrane emulsification. Typically this involves passing the mixture of the active ingredient and fatty component through a membrane having the desired pore size into a solution comprising the protein material and, optionally, the non-protein polymer and then vibrating the resulting mixture until an emulsion forms. The advantage of this process is that a narrower range of particle sizes is achievable than when standard emulsification techniques are used.

In the next step, known as "phase separation", two separate phases are created, namely, the continuous phase and the coacervate phase. The coacervate phase is generally based on the protein and, optionally, the non-polymer compound. This step is typically performed by modifying the physical environment of the mixture. Preferably phase separation is accomplished by modifying, preferably lowering, the pH to below the iso-electric point of the protein. If a non-protein polymer is present, the pH is preferably adjusted so that the positive charges on the proteins are neutralized by the negative charges on the non-protein polymer.

Phase separation may be induced by various other ways, in general by changing the physico-chemical environment of the solution. Depending on the kind of coacervation process (simple; complex) different ways of inducing phase separation can be applied, e.g. salting out or addition of a second high molecular weight component so as to induce entropic phase separation.

The temperature of the mixture is then reduced to below the gelling temperature of the protein. The determination of the gelling temperature of the gellable protein, preferably gelatine, is established, in part by experiment, the techniques of which are well known in the art.

In the final, optional step, cross-linking is performed to harden the colloid wall comprising the protein around internal phase. This step takes place spontaneously once the step of formation of a coacervate phase is induced.

Cross-linking is typically conducted at a temperature within the range of 5 to 40°C. Similarly, the pH during the cross-linking step is preferably adjusted to a level at which cross-linking can effectively be conducted. For example, if cross-linking is catalysed by the action of transglutaminase, the pH may preferably be adjusted to 3 to 7, more preferably 3.5 to 5.5 .

The breaking strength of the capsules according to the present invention is preferably high enough to provide a product that is more resistant to undesirable fracture upon storage. For instance, it is preferred that breaking strength value of the capsules is greater than 300g.cm⁻².

### End Products

The microcapsules according to the present invention can be used in many kinds of applications or consumer end products. Particular fields of interest are flavors and fragrances. Therefore, perfuming or flavoring compositions comprising microcapsules according to the invention, optionally together with other perfuming or flavoring co-ingredients, are also aspects of the present invention.

The invention is particularly useful in oral care products such as toothpastes and chewing gums.

Other suitable end products also include household care products, such as detergents. A further group of products where the invention can be applied is personal care products, such as cosmetics and shampoos.

### Examples

The invention will now be described with reference to the following examples, wherein the temperatures are indicated in degrees Celsius, values in tables denote % by weight, and the abbreviations have the usual meaning in the art, unless otherwise stated.

### Example 1

### Preparation of capsules according to the invention

A stock solution of gelatine (solution A) was prepared by mixing 180g of warm deionised water and 20g of gelatine (warm water fish gelatin, 200 Bloom, supplied by Weishardt) in a vessel until it was completely dissolved; the solution was then maintained at 40°C. A stock solution of gum Arabic (solution B) was prepared by mixing 180g of cold deionised water and 20g of gum Arabic (Efficacia®, from CNI) in a vessel until it was completely dissolved; the solution was then warmed and kept at 40°C.

A suspension of WS23 crystals in coco fat (solution C) was prepared by firstly heating at 40°C, 85g of hydrogenated Fat (Coco Fat - Margo Cocos) in a vessel until the fat was completely dissolved, adding 15g of cooling compound WS-23 (Millennium Specialty Chemicals, USA) and mixing with a high shear mixer until an homogeneous suspension was obtained. The solution was kept under vigorous agitation at 40°C.

105.4g of solution A was mixed with 70.3g of solution B in a vessel under gentle agitation (the gelatine/gum Arabic weight ratio being 1.5:1). The pH was adjusted to 4.6 with a 50% w/w aqueous lactic solution.

70.3g of solution C was slowly added to the gelatin/gum Arabic mixture and homogenised with a stirrer at 150 RPM for a period of 5 minutes, so as to reach an average droplet size of 500-1000µm.

The system was then diluted by the addition of 354.1g of warm deionised water, bringing the total hydrocolloid concentration to 3.4%w/w. The mixture was finally cooled to 20°C at a rate of 0.5°C.min⁻¹. The stirring speed was slightly decreased, and the pH adjusted to 4.5. Finally, 4.22g of transglutaminase (ACTIVA® WM supplied by Ajinomoto, Japan) was added to the mixture and cross-linking was allowed to proceed overnight at 20°C.

The result was an aqueous suspension of hard microcapsules.

### Example 2

### Preparation of further capsules according to the invention

A stock solution of gelatine (solution A) was prepared by mixing 180g of warm deionised water and 20g of gelatine (warm water fish gelatin, 200 Bloom, supplied by Weishardt) in a vessel until it was completely dissolved; the solution was then maintained at 40°C. A stock solution of gum Arabic (solution B) was prepared by mixing 180g of cold deionised water and 20g of gum Arabic (Efficacia®, from CNI) in a vessel until it was completely dissolved; the solution was then warmed and kept at 40°C.

The internal phase (solution C) was prepared by heating in a vessel at 60°C a mixture of 13.95g of coco fat (Margo Cocos) and 20.92g of hydrogenated palm oil (Stable flake P, Cargill) until the fat mixture was completely melted. 34.87g of mint oil (mint piperita, ex Firmenich) was then added and mixed to obtain an homogeneous solution. The solution was kept under gentle agitation at 45°C.

104.6g of solution A was mixed with 69.7g of solution B in a vessel under gentle agitation (the gelatine/gum Arabic weight ratio being 1.5:1). The pH was adjusted to 4.6 with a 50% w/w aqueous lactic solution.

69.7g of solution C is slowly added to the gelatine and gum Arabic mixture and homogenised with a stirrer at 150 RPM for a period of 5 minutes, so as to reach an average droplet size of 500-1000µm.

The system was then diluted by the addition of 355.9g of warm deionised water, bringing the total hydrocolloid concentration to 3.4%w/w. The mixture was finally cooled to 20°C at a rate of 0.5°C.min⁻¹. The stirring speed was slightly decreased, the pH adjusted to 4.5 and 4.22g of transglutaminase (ACTIVA® WM supplied by Ajinomoto) was added to the mixture. Cross-linking was allowed to proceed overnight at 20°C. The result was an aqueous suspension of hard microcapsules.

### Example 3a

### Preparation of Comparative Capsules

Coacervated capsules were prepared according to example 1 above except that the core material was a 50:50 w/w mixture of Neobee® (a medium chain triglyceride, ex Stepan) and mint oil (ex Firmenich). This core mixture has a melting temperature below 20°C. These capsules are referred to as Comparative Sample 1.

### Example 3b

### Preparation of Comparative Capsules

Coacervated capsules as described in publication WO-A1-2008/134908 were prepared in the manner set out in example 1 therein except that the Neobee® was replaced by limonene to provide a core component consisting of 20% beeswax and 80% limonene). This was in order to evaluate the stability of the capsules with a hydrophobic liquid active ingredient. These capsules are referred to as Comparative Sample 2.

### Example 4

### Comparison of Capsules in SDS solution

The coacervated capsules from examples 2 and 3 were then introduced into separate aqueous solutions comprising 1.5wt% sodium dodecyl sulphate ("SDS") and photographed at time 0 and 2 hours.

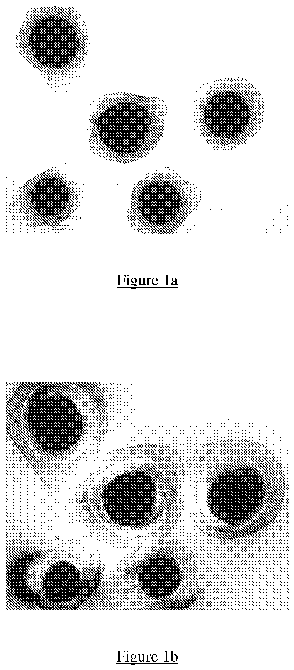

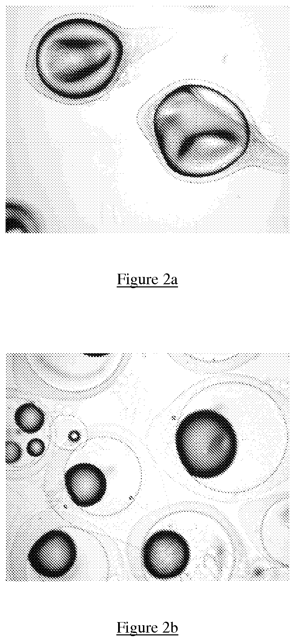

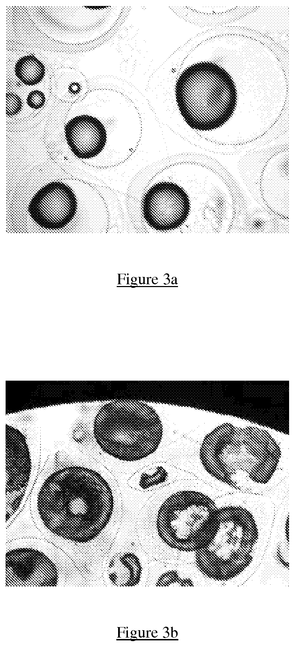

The results are shown in figures 1 to 3. Figure 1, which relates to a coacervated capsule according to the invention (example 2), demonstrates that there is substantially no loss of the active ingredient after 2 hours and that the core remains remarkably intact. By contrast, figure 2, which relates to Comparative Sample 1, shows a very significant loss of core material and Figure 3, which relates to Comparative Sample 2, shows that the core structure is often ruptured after during 2 hours.

## Claims

1. A coacervated capsule comprising:
(a) from 10 to 99% by weight of the capsule of a core comprising a mixture of (I) a fatty component comprising (i) hydrogenated oil or (ii) hydrogenated fat or (iii) cocoa butter or (iv) a mixture thereof, and (II) a material to be encapsulated comprising a flavor and/or fragrance material, the mixture having a Tm of between 30°C and 40°C such that it is a solid at 20°C, wherein the weight ratio of fatty component to material to be encapsulated is 50:50 and
(b) from 90 to 1% by weight of the capsule of a coating layer comprising essentially a protein, and optionally a non-protein polymer.

2. A capsule according to claim 1, wherein the flavor or a fragrance is a hydrophobic liquid.

3. A capsule according to any one of the preceding claims wherein the protein is gelatine.

4. A capsule according to any one of the preceding claims wherein the non-protein polymer is gum Arabic.

5. A process for the preparation of stable coacervated capsules comprising the step of:
(a) preparing a core mixture comprising:
(I) a fatty component comprising (i) hydrogenated oil or (ii) hydrogenated fat or (iii) cocoa butter or (iv) a mixture thereof and, and
(II) a material to be encapsulated comprising a flavor and/or fragrance material wherein the weight ratio of fatty component to material to be encapsulated is 50:50,
the core having a Tm of 30°C and 40°C such that it is a solid at room temperature;
(b) providing an aqueous solution comprising a protein and, optionally a non-protein;
(c) mixing the core mixture and the aqueous solution to form an emulsion or suspension;
(d) inducing phase separation such that the protein and, optionally a non-protein polymer, form a wall around the core mixture; and
(e) optionally cross-linking the wall.

6. A food composition comprising a coacervated capsule as claimed in any one of claims 1 to 4.

7. An oral care composition comprising coacervated capsules as claimed in any one of claims 1 to 4.

8. Use of a coacervated capsule as claimed in any one of claims 1 to 4 in a food, oral care, body care, skin care or home care product.

## Patentansprüche

1. Koazervierte Kapsel, umfassend:
(a) von 10% bis 99 Gew.-% der Kapsel von einem Kern, umfassend eine Mischung aus (I) einer Fettkomponente, umfassend (i) hydriertes Öl oder (ii) hydriertes Fett oder (iii) Kakaobutter oder (iv) eine Mischung daraus, sowie (II) ein Material, das eingekapselt werden soll, umfassend ein Geschmacks- und/oder Duftstoffmaterial, wobei die Mischung eine Tm zwischen 30 °C und 40 °C hat, so dass sie bei 20 °C ein Feststoff ist, wobei das Gewichtsverhältnis von Fettkomponente zu dem Material, das verkapselt werden soll, 50:50 beträgt, und
(b) von 90% bis 1 Gew.-% der Kapsel von einer Überzugsschicht, umfassend im Wesentlichen ein Protein und wahlweise ein Nicht-Proteinpolymer.

2. Kapsel nach Anspruch 1, wobei der Aromastoff oder ein Duftstoff eine hydrophobe Flüssigkeit ist.

3. Kapsel nach einem der vorgenannten Ansprüche, wobei das Protein Gelatine ist.

4. Kapsel nach einem der vorgenannten Ansprüche, wobei das Nicht-Proteinpolymer Gummi arabicum ist.

5. Verfahren zur Herstellung von stabilen koazervatierten Kapseln, umfassend die Schritte des:
(a) Herstellens einer Kernmischung, umfassend:
(I) eine Fettkomponente, umfassend (i) hydriertes Öl oder (ii) hydriertes Fett oder (iii) Kakaobutter oder (iv) eine Mischung daraus, und
(II) ein Material, das verkapselt werden soll, umfassend ein Geschmacks- und/oder Duftstoffmaterial, wobei das Gewichtsverhältnis von Fettkomponente zu dem Material, das verkapselt werden soll, 50:50 beträgt,
wobei der Kern eine Tm zwischen 30 °C und 40 °C hat, so dass er bei Raumtemperatur ein Feststoff ist;
(b) Bereitstellens einer wässrigen Lösung, umfassen ein Protein und wahlweise ein Nicht-Protein;
(c) Mischens der Kernmischung und der wässrigen Lösung, um eine Emulsion oder Suspension zu erzeugen;
(d) Einleitens einer Phasentrennung derart, dass das Protein und wahlweise ein Nicht-Proteinpolymer um die Kernmischung eine Wandung bilden; und
(e) wahlweisen Vernetzens der Wandung.

6. Nahrungsmittelzusammensetzung, umfassend eine koazervatierte Kapsel nach einem der Ansprüche 1 bis 4.

7. Mundpflegezusammensetzung, umfassend koazervatierte Kapseln nach einem der Ansprüche 1 bis 4.

8. Verwendung einer koazervierten Kapsel nach einem der Ansprüche 1 bis 4 in einem Lebensmittel-, Mundpflege-, Körperpflege-, Hautpflege- oder Haushaltsreinigungsprodukt.

## Revendications

1. Capsule coacervée comprenant:
(a) de 10 à 99% en poids de la capsule d'un noyau comprenant un mélange de (I) un composant gras comprenant (i) une huile hydrogénée ou (ii) une graisse hydrogénée ou (iii) du beurre de cacao ou (iv) un mélange de ceux-ci, et (II) un matériau à encapsuler comprenant un matériau d'arôme et/ou de fragrance, le mélange ayant une Tm entre 30°C et 40°C de sorte qu'il est un solide à 20°C, où le rapport en poids du composant gras sur le matériau à encapsuler est de 50:50 et
(b) de 90 à 1% en poids de la capsule d'une couche d'enrobage comprenant essentiellement une protéine et optionnellement un polymère non protéinique.

2. Capsule selon la revendication 1, dans laquelle l'arôme ou la fragrance est un liquide hydrophobe.

3. Capsule selon l'une quelconque des revendications précédentes, dans laquelle la protéine est une gélatine.

4. Capsule selon l'une quelconque des revendications précédentes, dans laquelle le polymère non protéinique est une gomme arabique.

5. Procédé pour la préparation de capsules coacervées stables comprenant les étapes de:
(a) préparation d'un mélange de noyau comprenant:
(I) un composant gras comprenant (i) une huile hydrogénée ou (ii) une graisse hydrogénée ou (iii) du beurre de cacao ou (iv) un mélange de ceux-ci, et
(II) un matériau à encapsuler comprenant un matériau d'arôme et/ou de fragrance, où le rapport en poids du composant gras sur le matériau à encapsuler est de 50:50,
le noyau ayant une Tm de 30°C et 40°C de sorte qu'il est un solide à la température ambiante;
(b) fourniture d'une solution aqueuse comprenant une protéine et optionnellement une non protéine;
(c) malaxage du mélange de noyau et de la solution aqueuse pour former une émulsion ou une suspension;
(d) induction d'une séparation de phases de sorte que la protéine et optionnellement un polymère non protéinique forment une paroi autour du mélange de noyau; et
(e) optionnellement réticulation de la paroi.

6. Composition alimentaire comprenant une capsule coacervée selon l'une quelconque des revendications 1 à 4.

7. Composition d'hygiène buccale comprenant des capsules coacervées selon l'une quelconque des revendications 1 à 4.

8. Utilisation d'une capsule coacervée selon l'une quelconque des revendications 1 à 4 dans un produit alimentaire, d'hygiène buccale, d'hygiène corporelle, de soin de la peau ou de soin domestique.
